# EUROPEAN PATENT APPLICATION

(11) **EP 4 321 199 A1**
(43) Date of publication of application: **14.02.2024**
(21) Application number: 21936035.1
(22) Date of filing: 08.04.2021
(51) Int. Cl.: A61M 25/06, A61M 25/092

(54) **MEDICAL INTRODUCER SHEATH WITH COUNTERMEASURE AGAINST INTRODUCTION OF AIR**

(71) Applicant: Togo Medikit Co., Ltd., Tokyo 113-0034 (JP)
(72) Inventor: ANDO, Hirokazu, Tokyo 113-0034 (JP); SHIBATA, Takahiro, Tokyo 113-0034 (JP)
(74) Representative: Grünecker Patent- und Rechtsanwälte PartG mbB
(86) International application number: PCT/JP2021/014923
(87) International publication number: WO 2022/215231

(57) **Abstract**

A medical introducer sheath having a steerable distal end portion and a proximal end portion fixed with a hub having a side port is provided with: an inner sheath elongated from the distal end portion to the proximal end portion and opened on the distal end portion and the proximal end portion, the inner sheath defining a main lumen in fluid communication with the side port; and an outer sheath running along an outer face of the inner sheath and defining one or more sub-lumens, each sub-lumen being elongated from the distal end portion and terminating at any position anterior to a proximal end of the inner sheath, the outer sheath covering the inner sheath throughout from the distal end portion to the proximal end portion and, at least at the proximal end portion, being in gas-tightly contact with or being formed in a unitary body with the inner sheath.

## Description

### TECHNICAL FIELD

The disclosure herein relates to an introducer sheath available for a medical treatment method such as catheter ablation, and in particular to a proximal end structure with measure against air intrusion in an introducer sheath with a wire-driven steerable tip.

### BACKGROUND ART

So-called catheter ablation is often carried out for the purpose of treatment of arrhythmia. In the catheter ablation, by using an introducer assembly having a sheath with an enough length to reach a patient's heart, a catheter is delivered to the heart from the patient's groin through a femoral vein for example and, by using its tip to apply radio frequency energy or other sources, a limited region in the heart is ablated. Similar assemblies are often used to treat varices, chronic pains or tumors.

To accurately guide the tip to affected areas, a sheath used in this type of treatment methods is so devised as to steer only its tip portion by hand operations. The proximal end portion of the sheath is, in addition, provided with a valve for taking in and out devices for treatments. If air happened to intrude into the heart, a serious problem would occur. Therefore the assembly is so structured as to connect with a syringe for the purpose of extracting the air.

Patent Literatures 1 through 3 disclose related arts.

### Citation List

### Patent Literature

| | |
|---|---|
| PTL 1: | PCT International Publication WO 2001/054763 A1 |
| PTL 2: | PCT International Publication WO 2007/136981 A1 |
| PTL 3: | Japanese Patent Application Laid-open No. 2020-062181 |

### SUMMARY OF THE INVENTION

Whereas bubbles in catheter ablation have been strictly managed as described already, it is known that intrusion of micro bubbles may rarely occur. How the hardly removable bubbles get therein have not been well understood but the present inventors have earnestly studied its origin and found out that this is due to a problem with the proximal end structure of the sheath, which conventional production processes would inherently create. The device described below has resulted from these studies.

According to an aspect, a medical introducer sheath having a steerable distal end portion and a proximal end portion for being fixed with a hub having a side port is provided with: an inner sheath elongated from the distal end portion to the proximal end portion and opened on the distal end portion and the proximal end portion, the inner sheath defining a main lumen in fluid communication with the side port; and an outer sheath running along an outer face of the inner sheath and defining one or more sub-lumens, each sub-lumen being elongated from the distal end portion and terminating at any position anterior to a proximal end of the inner sheath, the outer sheath covering the inner sheath throughout from the distal end portion to the proximal end portion and, at least at the proximal end portion, being in gas-tightly contact with or being formed in a unitary body with the inner sheath.

### BRIEF DESCRIPTION OF THE DRAWINGS

FIG. 1 is a schematic perspective view of an introducer sheath integrated in a handle.
FIG. 2 is a partial sectional plan view of the introducer sheath integrated in the handle.
FIG. 3 is a sectional plan view of the introducer sheath including a hub.
FIG. 4 is a cross sectional view of the introducer sheath, taken from a line IV-IV in FIG. 3.
FIG. 5 is a cross sectional view of the introducer sheath, taken from a line V-V in FIG. 3.
FIG. 6A is a longitudinal sectional view for illustrating production of a proximal end structure, which shows an inner sheath and sub-sheaths respectively supported by mandrels.
FIG. 6B is a longitudinal sectional view for illustrating production of the proximal end structure, which shows a state where the inner sheath and the sub-sheaths are covered with a braid.
FIG. 6C is a longitudinal sectional view for illustrating production of the proximal end structure, which shows a state where the inner sheath, the sub-sheaths and the braid are respectively cut properly.
FIG. 6D is a longitudinal sectional view for illustrating production of the proximal end structure, which shows a modified embodiment where modification is made as to the aspect of cutting the sub-sheaths.
FIG. 6E is a longitudinal sectional view for illustrating production of the proximal end structure, which shows a further modified example about modification as to the aspect of cutting the sub-sheaths.
FIG. 6F is a longitudinal sectional view for illustrating production of the proximal end structure, which shows an example where any portion except the rear ends of the sub-sheaths is cut.
FIG. 6G is a longitudinal sectional view for illustrating production of the proximal end structure, which shows an embodiment where modification is made as to the aspect of cutting the sub-sheaths or the braid.
FIG. 6H is a longitudinal sectional view for illustrating production of the proximal end structure, which shows a state of the introducer sheath after fusing the outer sheath in the example based on FIG. 6C.
FIG. 6I is a longitudinal sectional view for illustrating production of the proximal end structure, which shows a state of the introducer sheath after fusing the outer sheath in the example based on FIG. 6D.
FIG. 6J is a longitudinal sectional view for illustrating production of the proximal end structure, which shows a state of the introducer sheath after fusing the outer sheath in the example based on FIG. 6E.
FIG. 6K is a longitudinal sectional view for illustrating production of the proximal end structure, which shows a state of the introducer sheath after fusing the outer sheath in regard to the example based on FIG. 6F.
FIG. 6L is a drawing showing a state of the introducer sheath after fusing the outer sheath, which shows an example other than the example shown in FIG. 6K.
FIG. 6M is a longitudinal sectional view for illustrating production of the proximal end structure, which shows a state of the introducer sheath after fusing the outer sheath in the example based on FIG. 6G.

### DESCRIPTION OF EMBODIMENTS

Exemplary embodiments will be described hereinafter with reference to the appended drawings. Drawings are not necessarily to scale and therefore it is particularly noted that dimensional relations are not limited to those drawn therein.

A medical introducer sheath according to the present embodiment is mainly used for catheter ablation to treat heart diseases such as arrhythmia. It is of course available to other aims of treatment such as varices, chronic pains or tumors.

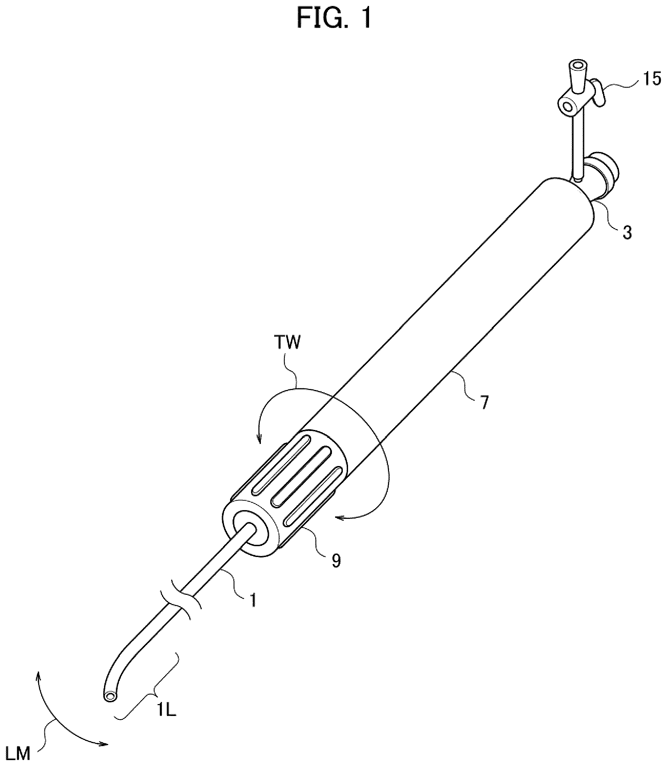

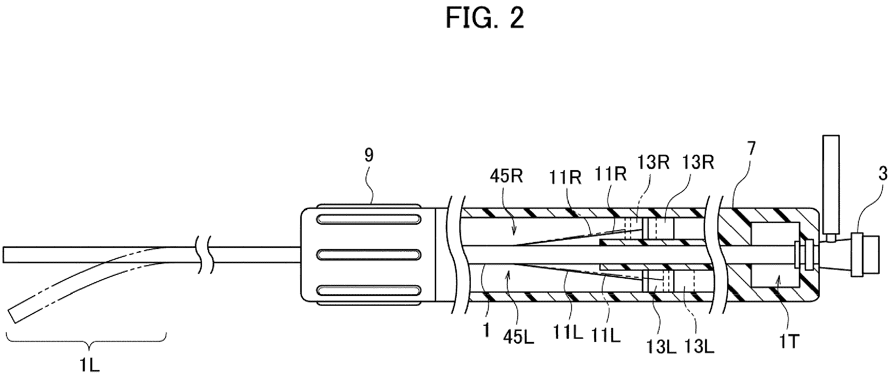

Referring mainly to FIGs. 1 and 2, a sheath 1 is generally a narrow elongated tube insertable into patient's veins or arteries, and is normally used in combination with a handle 7 for manual operation. Its distal end portion 1L is particularly flexible and thus allows itself to be steered in a way as the arrow LM shown in the drawings by turning a knob 9 at hand as the arrow TW.

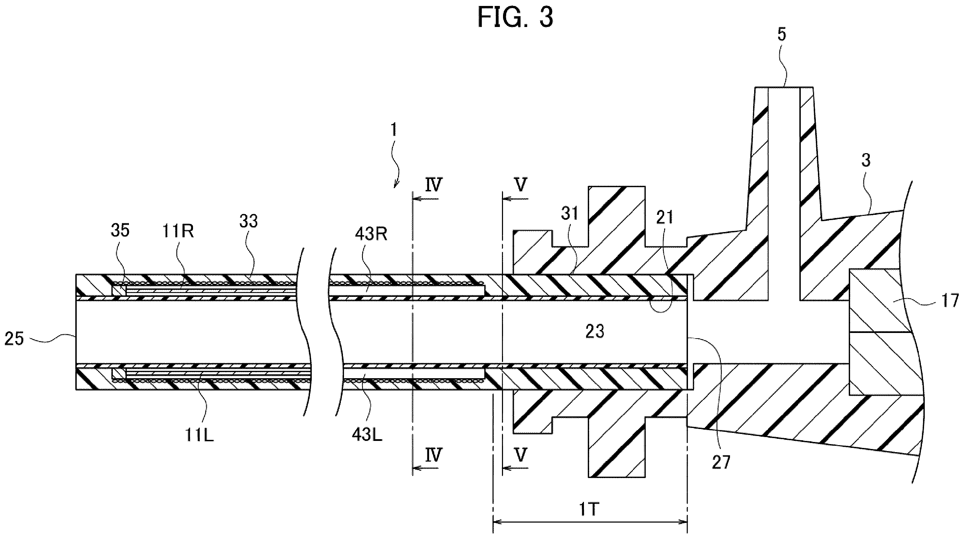

The entire length of the sheath 1 is enough to reach the patient's heart from the groin and is for example about 900 mm although of course it depends on the physical size of the patient. Its entirety is of a resilient resin so as to warp along the veins or the arteries and examples of the resin are, although not limiting, polytetrafluoroethylene, tetrafluoroethylene and perfluoroalkyl vinyl ether copolymer, aliphatic polyamide, polyether block amide, and a complex of two or more of these substances. The distal end portion 1L is made particularly thin in thickness or of a softer material so as to flexibly change in shape. A proximal end portion 1T housed in the handle 7 is made in gas-tight contact with the hub 3 and, as shown in FIG. 3, the hub 3 on its side face has a side port 5 in fluid communication with the interior of the sheath 1. The side port 5 is further connected to a three-way cock 15 and is thus served for suction by a syringe. Further, onto the proximal end of the hub 3, a valve 17 is made to fit, which is cleavable at its center for example, so that any device can be gas-tightly taken in and out of the assembly through the proximal end.

Referring mainly to FIG. 2, for the purpose of steering the distal end portion 1L, a pair of pull wires 11R, 11L for example may be used. Distal ends of the pull wires 11R, 11L are fixed to the distal end portions 1L and led through openings 45R, 45L on side faces of the sheath 1 to the exterior, and are connected to shuttle pieces 13R, 13L, respectively. The shuttle pieces 13R, 13L have a structural relation with the nob 9 via any proper cam mechanisms such as screws. If the nob 9 is turned in one direction, the left shuttle piece 13L moves rearward and the right shuttle piece 13R moves forward for example. As the pull wire 11L accordingly pulls the left side of the distal end portion 1L and the pull wire 11R loosens the right side, the distal end portion 1L deforms leftward as shown by chain lines in FIG. 2. Needless to say, if the nob 9 is turned in the counter direction, the distal end portion deforms rightward.

Referring to FIG. 3 in combination with FIG. 2, the sheath 1 is generally provided with an inner sheath 21 elongated throughout from the distal end portion 1L to the proximal end portion 1T, and an outer sheath 31 covering its outer periphery.

The inner sheath 21 is a narrow elongated tube formed of a very thin wall and defines a main lumen 23. The inner sheath 21 has an opening 25 on its distal end and has an opening 27 on its proximal end as well and the main lumen 23 spatially connects the opening 25 with the opening 27, thereby serving as a pathway through which any device such as a dilator passes. The opening 27 at the proximal end is, through the interior of the hub 3, in fluid communication with the side port 5, thereby serving for suction by a syringe.

While the outer sheath 31 covers the outer periphery of the inner sheath 21 and is preferably in close contact with its whole surface, it nonetheless defines sub-lumens 43R, 43L respectively corresponding to the pull wires 11R, 11L. The sub-lumens 43R, 43L do not open to the exterior although these distal ends reach the distal end portion 1L. Each sub-lumen 43R, 43L extends toward, but does not reach, the proximal end portion 1T and terminates at any position anterior thereto. Meanwhile the proximal end portion 1T is illustrated in FIG. 3 to be longer than a portion fitting into the hub 3 and its length is about from several mm to several cm but the present embodiment is not necessarily limited thereby. The sub-lumens 43R, 43L may be elongated to around the proximal end face as long as they do not reach the end face to form openings.

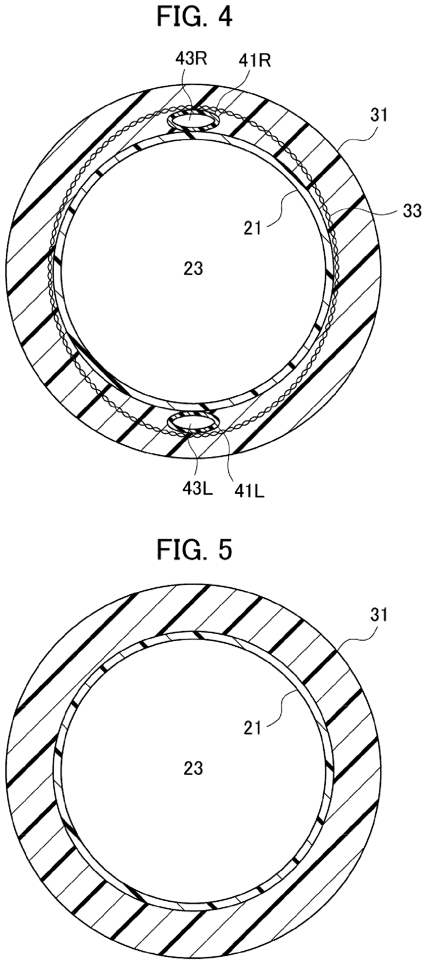

Referring to FIG. 4 in combination with FIGs. 2, 3, to define the sub-lumens 43R, 43L, the sheath 1 may be provided with sub-sheaths 41R, 41L embedded in the outer sheath 31. The sub-sheaths 41R, 41L are likewise narrow elongated tubes formed of tetrafluoroethylene and perfluoroalkyl vinyl ether copolymer for example, and structurally support the sub-lumens 43R, 43L so as not to collapse.

Referring mainly to FIG. 3, respective distal ends of the pull wires 11R, 11L are fixed to the distal end portion 1L. Although they may be directly fixed to the inner sheath or the outer sheath 31, they may be commonly fixed to a ring 35 and the ring 35 may be fixed to the inner sheath 21 and/or the outer sheath 31. The pull wires 11R, 11L are guided rearward from the distal end portion 1L through the sub-lumens 43R, 43L respectively, particularly inside the sub-sheaths 41R, 41L if any. As described already, the outer sheath 31 has openings 45R, 45L anterior to the proximal end portion 1T, which are respectively in communication with the sub-lumens 43R, 43L. The pull wires 11R, 11L are led respectively through these openings 45R, 45L out of the outer sheath 31, and are thus respectively connected to the shuttle pieces 13R, 13L.

The sheath 1 may be as well provided with a braid 33 embedded in the outer sheath 31. The braid 33 is for example a tubular fabric in which very thin wires of a stainless steel for example are woven together, and covers the inner sheath 21, or the sub-sheaths 41R, 41L along with the inner sheath 21 as well. The braid 33 is elongated along the inner sheath 21 and its front end and rear end may be generally aligned with both ends of the sub-sheaths 41R, 41L or either may be made longer. In any case, the braid 33 does not reach the proximal end portion 1T of the sheath 1, but terminates at any place anterior to the proximal end portion 1T.

Referring to FIG. 5 in combination with FIGs. 2, 3, as the sub-lumens 43R, 43L and the braid 33 commonly terminate at any place anterior to the proximal end portion 1T, they do not appear on the section around the proximal end portion 1T. The outer sheath 31 is directly in gas-tight contact with, or forms a unitary body with, the inner sheath 21. This structure prevents air leakage through around the sub-lumens 43R, 43L or the braid 33. Any portions of the sub-sheaths 41R, 41L may, however, be contained in the proximal end portion 1T as long as the sub-lumens 43R, 43L do not reach the proximal end of the sheath 1.

Although details will be described later, as the inner sheath 21, the sub-sheaths 41R, 41L and the outer sheath 31 are mutually in gas-tight contact or form a unitary body by means of fusion bonding for example, they might not be necessarily recognized separately under microscopic observation for example. The mutual fusion could give rise to misleading observation as if these members form a unitary body.

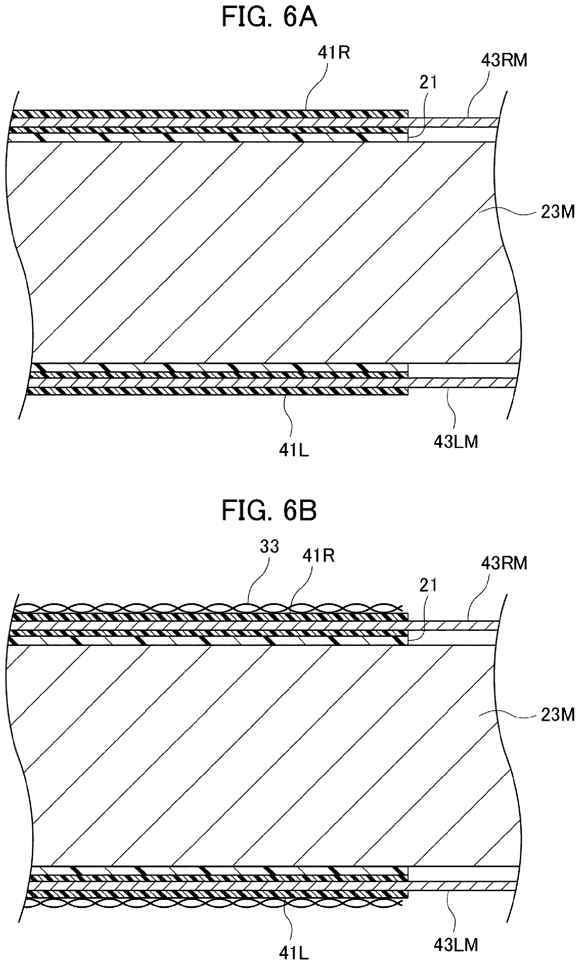

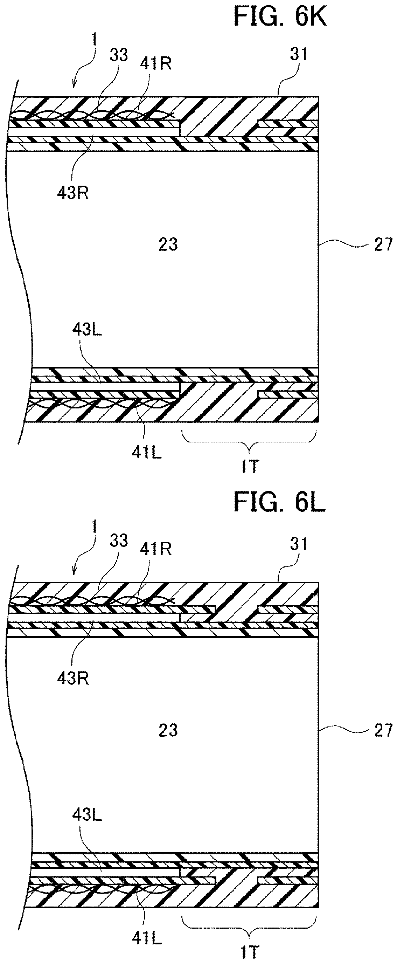

Referring to FIG. 6A through FIG. 6M, an example of steps for producing the sheath 1 will be described below, as to the production of the proximal end structure particularly.

The inner sheath 21 and the sub-sheaths 41R, 41L may be produced by extrusion molding for example. Referring to FIG. 6A, the inner sheath 21 and the sub-sheaths 41R, 41L are prepared in a state where mandrels 23M, 43RM, 43LM, which may be metal wires for example, pass through these members. The sheaths and the mandrels may be independently prepared and thereafter the latter may be made to pass through the former, or mandrels used for producing the sheaths may be left there and used as-is. In this state, three sheaths 21, 41R, 41L are drawn straight and side-by-side with having the inner sheath 21 between the sub-sheath 41R, 41L.

Referring to FIG. 6B, next the braid 33 is made to cover the sub-sheath 41R, 41L along with the inner sheath 21. These proximal ends may be aligned with each other, or misaligned to some degree.

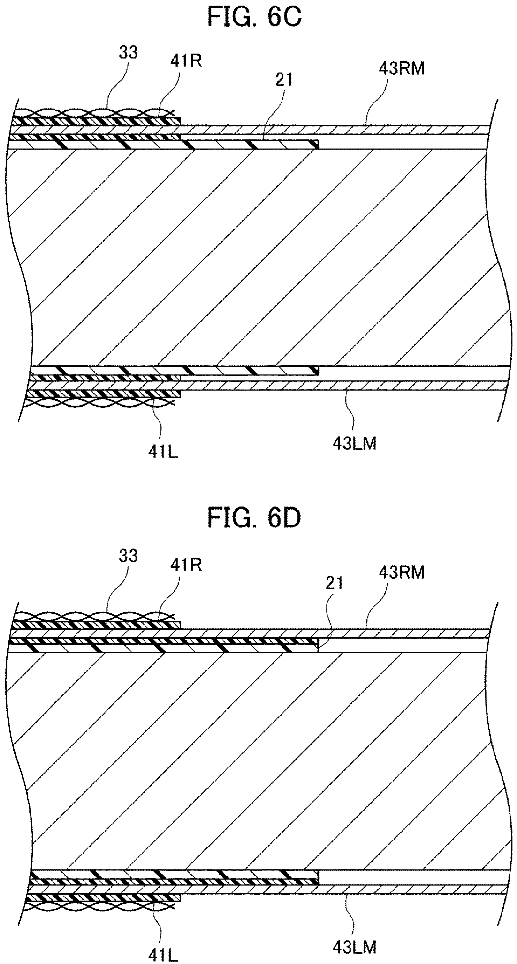

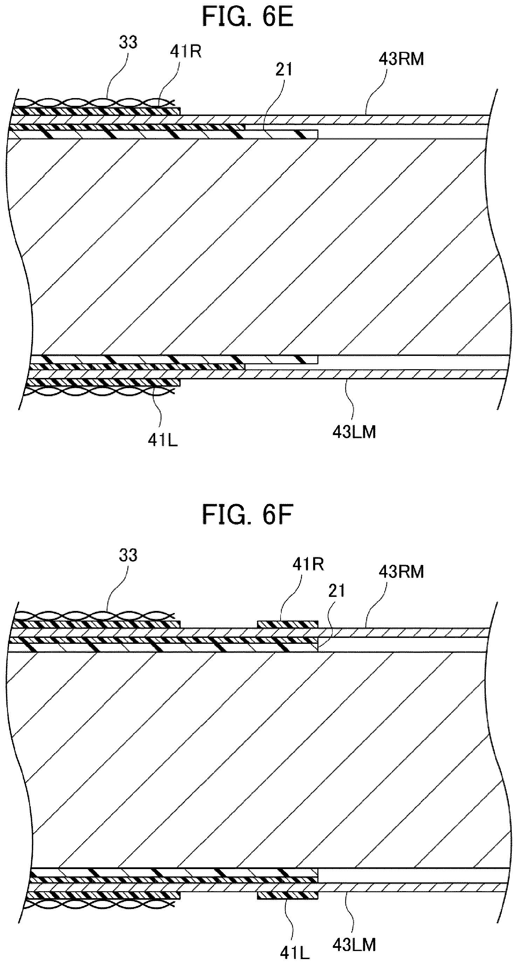

Referring to FIG. 6C, next from 10 mm to several tens mm of the proximal ends of the sub-sheaths 41R, 41L and the braid 33 are cut out so that these ends terminate anterior to the proximal end of the inner sheath 21. Then a small length of the proximal end of the inner sheath 21 may be cut out. As well, the whole circumferences of the sub-sheaths 41R, 41L may be cut out but, as shown in FIG. 6D, only these outer sides may be cut and other sides in contact with the inner sheath 21 may be left there. Alternatively, as shown in FIG. 6E, the sides in contact with the inner sheath 21 may be also cut but positions of cutting may differ from those of the outer sides.

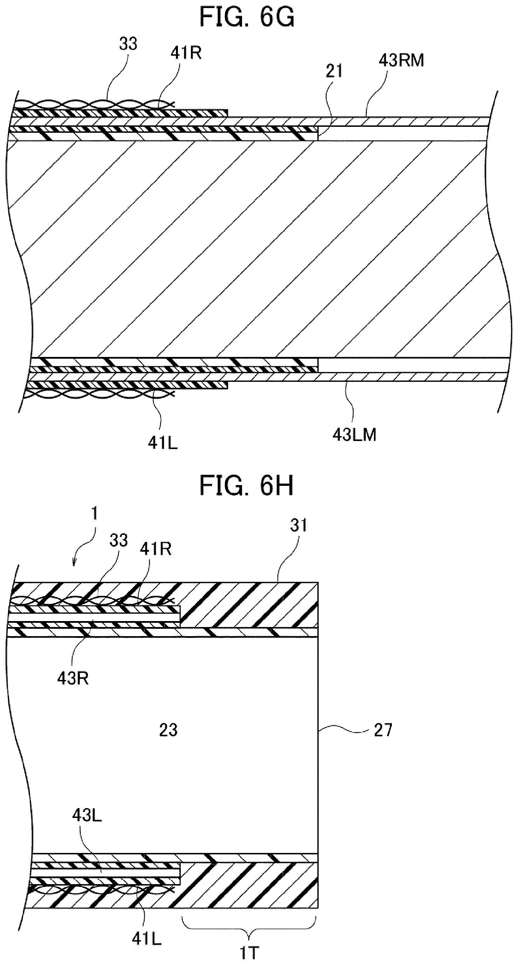

Still alternatively, as shown in FIG. 6F, any positions at the outer sides other than the end portions may be cut. Further, the position for cutting in the braid 33 may be, as shown in FIG. 6G, different from the positions for cutting the sub-sheaths 41R, 41L.

In any case, the mandrels 43RM, 43LM are removed and instead the pull wires 11R, 11L are introduced therein and, along with the ring 35, fixed to the inner sheath 21. Then, to close the proximal ends of the sub-sheaths 41R, 41L, any filler, adhesive or such may be injected therein respectively.

Referring to FIG. 6H through FIG. 6M, the outer sheath 31 is made to further cover the inner sheath 21, the sub-sheaths 41R, 41L and the braid 33, and the whole is bound together by means of heating to carry out fusion bonding or such. The outer sheath 31 may be put over the braid 33, or the braid 33 may be covered with a mold in advance and any material may be thereafter heated and injected into the mold to form the outer sheath 31 over it. In any case, the outer sheath 31, during heating, becomes sufficiently fluent and infiltrates into the braid 33 and forms a unitary body with it.

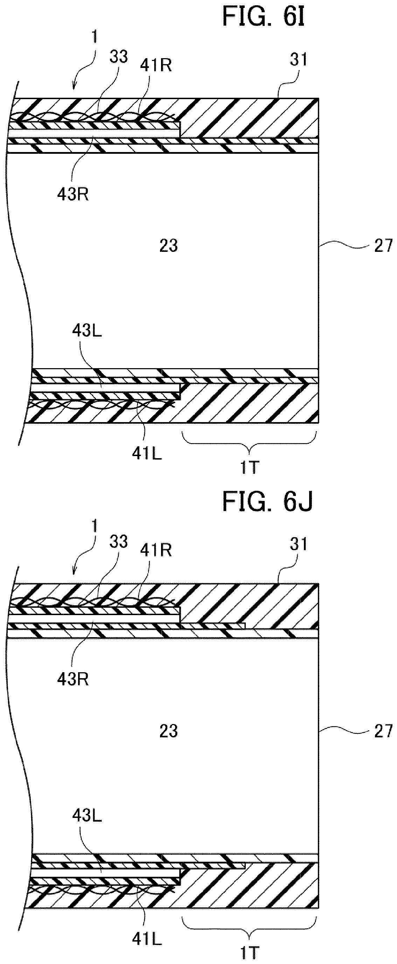

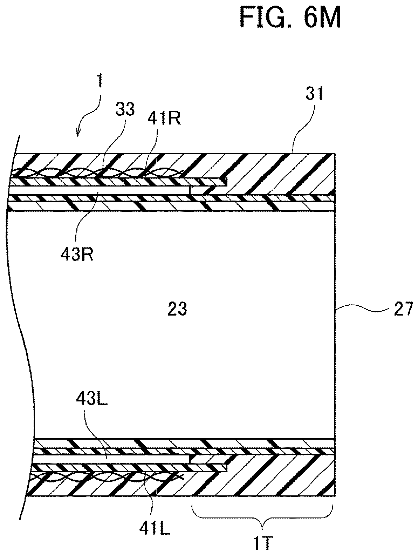

In a case where the sub-sheaths 41R, 41L are cut so as to terminate at any positions anterior to the proximal end of the inner sheath 21 as shown in FIG. 6C, 6E or 6G, the sub-lumens 43R, 43L necessarily terminate at the positions anterior to the proximal end of the sheath 1 as shown in FIG. 6H, 6J or 6M and therefore the sub-lumens 43R, 43L are gas-tightly sealed at the proximal end portion 1T. In a case where only the radially outer sides of the sub-sheaths 41R, 41L are cut as shown in FIG. 6D, the outer sheath 31 at its proximal end has portions not directly in contact with the inner sheath 21 as shown in FIG. 6I but the outer sheath 31 can nonetheless seal the sub-lumens 43R, 43L gas-tightly and is, insofar, in gas-tightly contact with the inner sheath 21. In a case where any positions at the outer sides other than the end portions are cut as shown in FIG. 6F, the outer sheath 31, when heated, can flow through these position into the sub-lumens 43R, 43L and therefore the sub-lumens 43R, 43L terminate at the positions anterior to the proximal end of the sheath 1 and the gas-tightness is retained at the proximal end portion. Further as described already, any filler or adhesion may gas-tightly close the sub-lumens 43R, 43L as well as the outer sheath 31.

The outer sheath 31 and the inner sheath 21 may be aligned together and then the latter may be covered with the former, or these members may be so cut, in advance of bonding, but instead they may be in advance bonded together and thereafter cut so that the end faces are aligned. By removing the mandrel 23M, the sheath 1 can be obtained.

Referring again to FIG. 3, the proximal end portion 1T of the sheath 1 is inserted into the hub 3 and its very end face is made to abut on a corresponding face in the hub 3. The proximal end portion 1T is, by any means such as press-fitting, adhesion or bonding, gas-tightly bonded with the hub 3.

The production method as described above is no more than an example and may be properly modified or changed.

According to the prior art, because a braid and sub-sheaths are, as being aligned, bonded with an outer sheath, the sub-lumens necessarily open on the proximal end face of the sheath and further the braid is exposed on the proximal end face. As the proximal end face is made to abut on the internal face of the hub and thus closed, nonetheless such a structure generally causes no issues. The studies by the present inventors have, however, revealed that such closure is not sufficient and, in a case where the interior of the main lumen is given negative pressure, bubbles may be sucked in through the openings through which the pull wires are taken out, and through the sub-lumens, and further through minute gaps between the proximal ends and the internal face of the hub. The negative pressure can be applied actually by the syringe, or rarely raised by heart beats. According to the present embodiments, because the sub-lumens 43R, 43L and the braid 33 commonly terminate at positions anterior to the proximal end of the outer sheath 31 and therefore do not reach its proximal end face, any pathways for sucking bubbles are excluded and the contact between the proximal end face of the sheath 1 and the internal face of the hub would pose no issues. It is apparent that small possibility of air intrusion can be prevented.

Although certain exemplary embodiments are described above, modifications and variations of the embodiments will occur to those skilled in the art, in light of the above teachings.

### Industrial Applicability

A medical introducer sheath with measure against air intrusion is provided.

## Claims

1. A medical introducer sheath having a steerable distal end portion and a proximal end portion for being fixed with a hub having a side port, the introducer sheath comprising:
an inner sheath elongated from the distal end portion to the proximal end portion and opened on the distal end portion and the proximal end portion, the inner sheath defining a main lumen in fluid communication with the side port; and
an outer sheath running along an outer face of the inner sheath and defining one or more sub-lumens, each sub-lumen being elongated from the distal end portion and terminating at any position anterior to a proximal end of the inner sheath, the outer sheath covering the inner sheath throughout from the distal end portion to the proximal end portion and, at least at the proximal end portion, being in gas-tightly contact with or being formed in a unitary body with the inner sheath.

2. The introducer sheath of claim 1, further comprising:
sub-sheaths embedded in the outer sheath and respectively defining the sub-lumens.

3. The introducer sheath of claim 1, further comprising:
wires fixed to the distal end portion and respectively passing through the sub-lumens,
wherein the outer sheath includes openings anterior to the proximal end portion and respectively in communication with the sub-lumens, and the wires are respectively led through the openings out of the outer sheath.

4. The introducer sheath of claim 1, further comprising:
a braid embedded in the outer sheath and elongated along the inner sheath to terminate at any place anterior to the proximal end portion.
